# EUROPEAN PATENT APPLICATION

(11) **EP 1 473 000 A1**
(43) Date of publication of application: **03.11.2004**
(21) Application number: 03380101.0
(22) Date of filing: 28.04.2003
(51) Int. Cl.: A61F 5/41

(54) **Genitalia external prothesis for treatment of male external genitalia hypoplasla**

(71) Applicant: Gomez de Diego, Eduardo, 28013 Madrid (ES)
(72) Inventor: Gomez de Diego, Eduardo, 28013 Madrid (ES)

(57) **Abstract**

The invention describes a device designed for the treatment of the external male genitalia hipoplasia, creating a growth of tissues and a correction of existing curvatures.

The invention's device is unique for using an external genitalia fixation (1; 13) associated to a diametric system (5) and to a gland protection system (15). The system of external genitalia inferior fixation (1) and superior (13) produces on the external male genitalia a traction force. The diametric system (5) creates on the male genitalia a tissue expansion. The gland protection system (15) avoids possible cuts, this allows a long term use of the device, free of discomfort. The device's user can wear it for many months, during a few hours a day. This way, the chance of a tissue expansion plus an elongated expansion in time are fundamental for the external male genitalia hipoplasia treatment.

## Description

The present invention relates to a method of generating a permanent elongation of the male genitalia. The invention moreover relates to a device for performing the method, said device comprising a substantially ring-shaped element to be fitted proximally on the male genitalia and connecting elements which are secured at their one end to the ring-shaped element by means of hinge elements, and which substantially extend along the male genitalia to a region around the glands, where the connecting elements are provided with holding means for holding the distal end of the male genitalia.

Since 1905 it has been known to use tissue traction for stretching parts of the body, and in 1957 Neuman performed tissue expansion in practice. Traction of fingers was first described by the American Cowen who at the annual U.S. Congress of Orthopedic Surgery in 1977 spoke about stretching fingers to normal length in children who had been born with too short fingers. On the basis of his clinical tissue distractions Cowen found that blood vessels and "all" other tissues on the extremity are stretched, provided that the rate of traction does not exceed the "rate of nerve regeneration".

Animal experiments have shown that the epidermis responds to constant expansion/tension by increased cell division activity in the basal layer, a process which reaches its maximum within 24-48 hours and normalizes within 6 days.

Animal experiments have shown that expansion of the dermis reduces its thickness slightly. Normalization of the thickness of the dermis after 2 years expansion has been reported.

Devices of the typed mentioned in the opening paragraph are known for use as aids during intercourse, i.e. for shorter periods of time.

WO 84 91284, e.g. , discloses a device of the type mentioned in the opening paragraph for supporting the male genitalia at a fixed angle from the body during intercourse. Prior to mounting the device, it must be adjusted to fit the length of the male genitalia concerned. The feasibility of subsequent adjustment of the length of the device is not mentioned in the document. EP 370 932 discloses another device for buttressing the male genitalia.

It is common to the above-mentioned devices that they are exclusively adapted to straighten the male genitalia. Therefore, the devices are not adapted to be frequently adjusted in length and are moreover just adapted to be worn during intercourse.

DE 166168 discloses a device for straightening the urethra to thereby remedy a disorder, such as contracting scars which bend the male genitalia and causes painful urination. The device consists of a metal rod which extends along the male external genitalia and is adapted to support the male genitalia. The device is constructed to fit the male genitalia concerned and does not stretch it, but merely straightens it for relief of pains, e.g. in connection with urination. The device, like the invention device, compromises hinge elements at the proximal end of the male genitalia, so that the device can be worn for an extended period of time.

The invention describes a device designed for the treatment of the external male genitalia hipoplasia, creating a growth of tissues and a correction of existing curvatures.

The invention's device is unique for using an external genitalia fixation associated to a diametric system and to a gland protection system. The system of external genitalia fixation produces on the external male genitalia a traction force. The diametric system creates on the male genitalia a tissue expansion. The gland protection system avoids possible cuts, this allows a long term use of the device, free of discomfort. The device's user can wear it for many months, during a few hours a day. This way, the chance of a tissue expansion plus an elongated expansion in time are fundamental for the external male genitalia hipoplasia treatment.

These and other characteristics and advantages of the invention will be better explained in a following detailed description that follows preferred steps, given as an illustrated example and not limited, referring to the attached drawings, in which:
Fig. 1 shows the device in its entirety, fitted on the genitalia masculine organ.
Fig. 2 shows the device with the secondary elements of the internal fixation added in a microscope way to itself, place on the male genitalia, in a posterior vision.
Fig. 3 shows the different secondary elements of the internal fixation system available.
Fig. 4 shows a vision of the internal fixation system and of the dynamometric system
Fig. 5 shows the silicone ring and the gland protection system

To obtain de detailed description of the preferred execution of the current invention, it will be referred to the annex drawings figures, and to the different parts and sections in which have been represented.

The invention device is made of a system of external genitalia fixation, of an internal fixation system, of a gland protection system and of a tissue expansion dynamometric system.

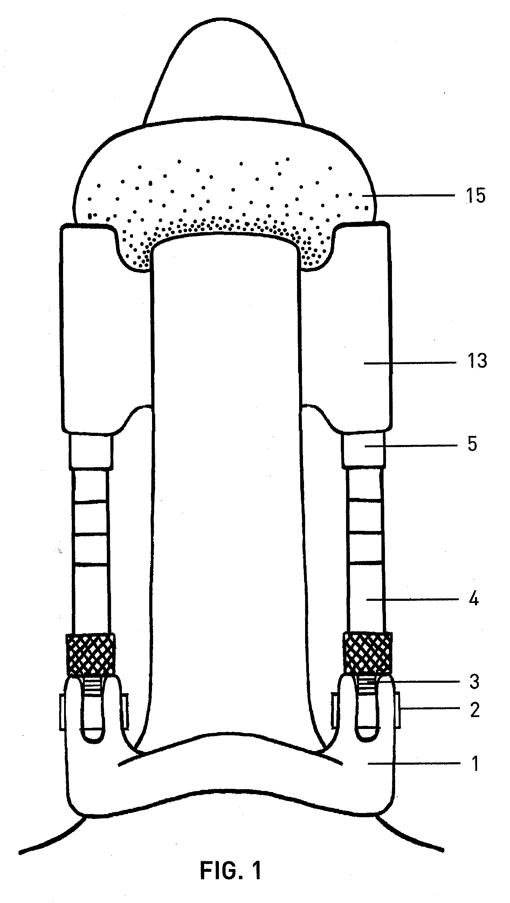

This way, looking at figure 1, you can see the invention device in its entirety, placed on the external male genitalia.

As shown in figure 1 the external fixation system is formed by an inferior element 1 and an a superior element 13. The inferior element of the external genitalia fixation 1 is an angular base that is placed in the pubic area of the user and around his internal angular area the device is placed. In the inferior element of the external genitalia fixation system 1 is assembled two rounding elements 2 situated each one in each side.

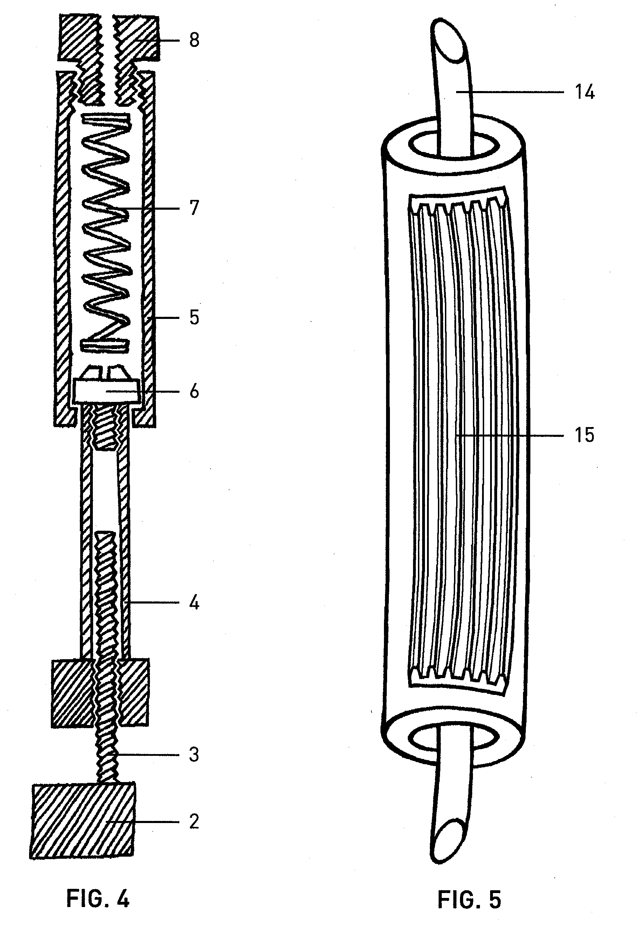

As observed in figure 1 and figure 4 each rounding tool 2 is united to a tubular set made of a screw 3, a screwdriver with inferior head 4, a metal tube 5 and a superior screw 8, making all these pieces the internal fixation of the external genitalia fixation 1;13

In figure 1 you can see like each internal fixation system fits in the superior element of the external genitalia fixation 13. The superior of the external genitalia fixation 13 is a plastic support with two holes to introduce the final extreme sides of each internal fixation system.

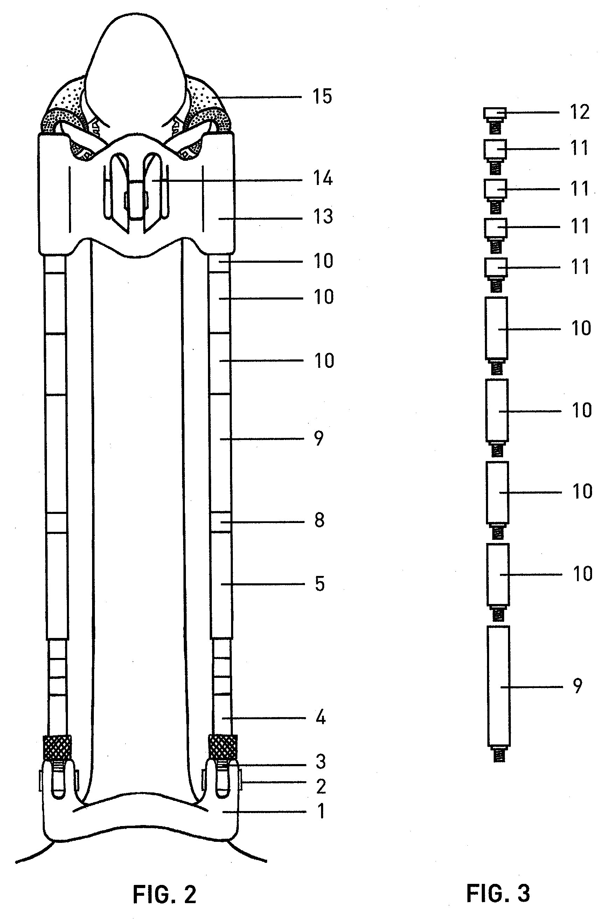

In the Figure 1 and Figure 2 it is observed that the superior element of external genitalia fixation 13 secures the gland to the device, helped by a silicone ring 14 whose extremes fit in the superior part of the superior element of the external genitalia fixation 13. In the silicone ring 14 is placed the gland protection system 15.

In figure 2 the invention device is shown with the secondary elements 9; 10 of the internal fixation system added telescopically from tool 8. Mentioned secondary elements 9;10 are necessary to adapt the length of the male genitalia to the external fixation system 1;13 In mentioned figure 2 you can see the invention's device placed on the male genitalia, in a posterior view.

Figure 3 shows the different secondary elements available 9; 10; 11; 12 of the internal fixation system. Mentioned secondary elements 9; 10; 11; 12 have a screw shape, varying in number and size.

Figure 4 shows a vision of the internal fixation and of the dynamometric system. Each rounding piece 2 unites to a tubular set made by a metal bar 3, an adjustable screw with inferior top 4, a metal tube 5 and a superior adjustable screw 8, making all these pieces the internal fixation system of the external genitalia fixation 1; 13. The dynamometric system is made of a cylinder 5, an inferior internal screw 6, a spring 7 and a superior adjustable screw 8.

Figure 5 shows the silicone ring 14 and the gland protection system 15. The gland protection system 15 is placed on the silicone ring 14, that is placed in the glans sulcus area. The gland protection system 15 is made by cushioning and traction elements.

It is not necessary to extend the contents of this description so an expert in this field can understand the goals and advantages originated in this invention, and so to develop and take into practice its function.

Nonetheless, it should be understood that the invention has been described according to a preferred handling of its own, which can be exposed to modifications which doesn't mean that the basis of the invention would be modified, that the shape could be changed, nor the size and the elements of fabrication.

## Claims

1. A device for the treatment of external male genitalia hipoplasia, that is made of an external genitalia fixation system (1; 13), of an internal holding system (2; 3; 4; 5; 8), of a dynamometric system of tissue expansion (5; 6; 7; 8 ) and of a gland protection system (15), being the external system of fixation made of an inferior element (1) and a superior element (13), being the inferior element (1) a angular base that is placed on the pubic area of the user so that its annular area fits the male genitalia and in which two rounding elements are assembled (2) situated on each side, uniting each rounding piece (2) to a tubular set (3; 4; 5; 8), forming both the internal fixation system (2; 3; 4; 5; 8) of the external genitalia system (1; 13), having such internal fixation system (2; 3; 4; 5; 8) secondary elements (9; 10; 11; 12) added telescopically to the final piece (8) of the tubular set (3; 4; 5; 8), being inserted inside the tubular set (3; 4; 5; 8) the dynamometric system (5; 6; 7; 8) formed by a embolo.

2. Such embolo is a cylinder (5), an inferior screw (6), a spring (7) and a superior screw (8), fitting the final ends of each tubular set (3; 4;5;8) in the superior element of the external male genitalia fixation system (13), of which is a plastic support that stabilizes the tubular sets (3; 4; 5;8), being such superior element of the external male genitalia fixation system (13) where the gland is placed to the device, helped by a silicone ring (14) which ends are fitted in the posterior side of the superior element of the external male genitalia fixation system (13), adding to the silicone ring the gland protection system (15).
